# EUROPEAN PATENT SPECIFICATION

(11) **EP 2 255 751 B1**
(45) Date of publication and mention of the grant of the patent: **20.03.2013**
(21) Application number: 10164319.5
(22) Date of filing: 28.05.2010
(51) Int. Cl.: A61F 2/16

(54) **Intraocular lens injection instrument**
Einspritzinstrument für eine Intraokularlinse
Instrument d'injection de lentille intraoculaire

(30) Priority: 29.05.2009 JP 2009131354
(43) Date of publication of application: 01.12.2010
(73) Proprietor: NIDEK CO., LTD, Gamagori-shi Aichi 443-0038 (JP)
(72) Inventor: Nagasaka, Shinji, Gamagori-shi Aichi 443-0038 (JP)
(74) Representative: Prüfer & Partner GbR European Patent Attorneys

(56) References cited:
- EP-A1- 1 972 306
- EP-A2- 1 360 944
- WO-A1-2008/029498

## Description

### Technical Field

The present invention relates to an intraocular lens injection instrument for injecting an intraocular lens in an eye.

### Background Art

As one of operative treatments for cataract, heretofore, there has generally been used a method in which a lens is removed from a patient's eye and then a foldable intraocular lens (IOL) is injected in place of the lens. Such IOL includes a foldable optic part and support parts for supporting the optic part in the eye. For injection of the IOL, an IOL injection instrument called an injector is used to inject the IOL in a folded state into the eye so that an incision formed in the eye is as small as possible. As such injector, there is recently known an instrument serving to not only simply inject an IOL in an eye but also storage the IOL (for example, see Patent Literature 1). This instrument retains the IOL by use of a member for storing the IOL in a fixed state in the injector so that the IOL can be pushed out by detachment of that member in use. Furthermore, many foldable IOLs made of acrylate resin have adhesive properties because of its resin property. Accordingly, the IOL is liable to adhere to the inside of the injector due to long-term storage and could not be pushed out smoothly. In view of such circumstances, the injector also used to store an IOL as described in Patent Literature 1 has a mechanism for fixing the IOL under no stress during storage and moving the IOL to a position where the IOL is allowed to be pushed by a push-out mechanism in use.

Document WO 2008/029498 A1 discloses an intraocular lens inserting instrument, wherein a tool body of generally tubular shape includes a placement part having a placement surface for placement of the intraocular lens. A carrying member is attached from an outside to a placement surface formation site. Leg and main body support parts of the carrying member project through through-holes formed in the placement surface to support the intraocular lens. The carrying member can be detached from the placement surface, such that the intraocular lens is placed on the placement surface.

### Citation List

### Patent Literature

Patent Literature 1: JP 2006-181269A (WO 2006/070561A1)

### Summary of Invention

### Technical Problem

In the above injector, to facilitate push-out of the IOL, a viscoelastic material is usually supplied to the vicinity of the IOL and onto an injector push-out path just before use. In the injector in Patent Literature 1, however, a space or clearance between a position (place) for storing the IOL and a position (place) for pushing out the IOL in use is narrow. Thus, the viscoelastic material is less hard to be injected to a region under the IOL. On the other hand, in a configuration that the IOL is stored in a position sufficiently apart from the push-out position to keep a distance between the IOL and a setting stage, the viscoelastic material can be injected appropriately. However, due to the presence of the viscoelastic material injected before the IOL is moved to the push-out position, the IOL is less likely to be placed appropriately in the push-out position.

The present invention has been made in view of the circumstances and has a purpose to provide an IOL injection instrument capable of appropriately injecting a viscoelastic material to the vicinity of an IOL so that the viscoelastic material is applied to the IOL, and also placing the IOL in a push-out position without causing displacement.

### Solution to Problem

To achieve the above purpose, the present invention provides an intraocular lens injection instrument for storing a soft intraocular lens (IOL) including an optic part made of acrylate resin having viscosity in a housing of the instrument, and feeding out the IOL from the housing through a front end of the instrument by use of a push rod of the instrument to inject the IOL into a patient's eye, **characterized in that** the instrument comprises a storage part provided with a pair of holding stands arranged opposite to each other in a front-rear direction along a movement axis of the push rod to hold the optic part, the holding stands including upper surfaces on which the optic part is to be placed and having a predetermined height to place the optic part above and out of the movement axis during storage, the upper surfaces have a surface roughness of arithmetical mean roughness Ra 6.3 or less so that the optic part is allowed to adhere to the upper surfaces by the viscosity of the optic part and the surface roughness of the upper surfaces, and the storage part is vertically movably attached to the housing so that the optic part is placed above and out of the movement axis when the storage part is positioned upside and the optic part is placed on the movement axis when the storage part is positioned downside.
Further developments of the present invention are given in the dependent claims.

### Advantageous Effects of Invention

According to the invention, a viscoelastic material can be appropriately injected to the vicinity of an IOL and applied to the IOL. Furthermore, the IOL can be placed in a push-out position without causing displacement.

### Brief Description of Drawings

FIG. 1 is an external view of an IOL;
FIG. 2 is a schematic configuration view of an IOL injection instrument in an embodiment;
FIG. 3 is a sectional view taken along a line A-A in FIG. 2;
FIG. 4 is a perspective view of a storage member; and
FIG. 5A and 5B are enlarged sectional views of a setting part and its surroundings.

### Description of Embodiments

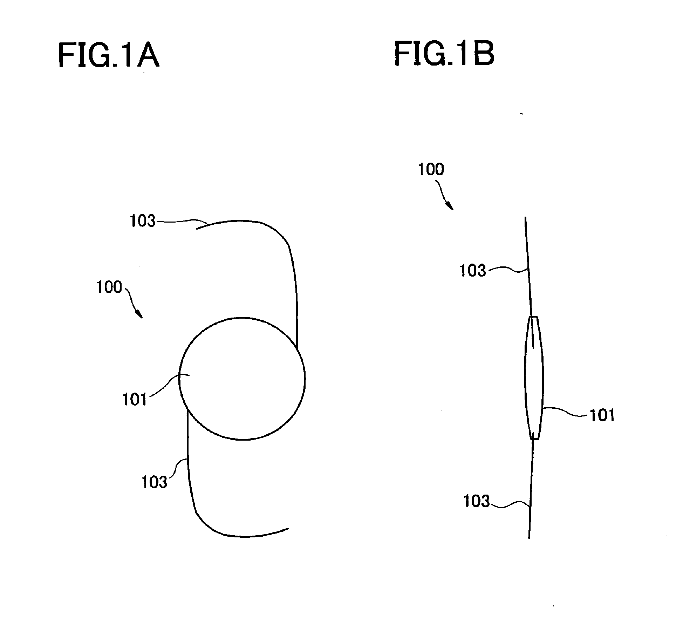

A detailed description of a preferred embodiment of the present invention will now be given referring to the accompanying drawings. FIG. 1 is a configuration view of an IOL 100 previously set in an IOL injection instrument in this embodiment. The IOL 100 includes an optic part 101 having predetermined refractive power and a pair of support parts 103 for fixedly supporting the optic part 101 in a patient's eye.

The optic part 101 in this embodiment has such predetermined adhesive properties as to produce predetermined adhesive power (described later) when a storage member mentioned later and the optic part 101 contact with each other. The optic part 101 is made of a soft, foldable IOL base material such as acrylate resin. This IOL base material can be obtained by combining one or more kinds of monomers constituting a soft material. Alternatively, one or more kinds of monomers constituting a hard material may be added to adjust the hardness (softness) of the base material. Concrete examples of the monomers constituting the soft material may include acrylic ester such as methylmethacrylate, ethylacrylate, propylacrylate, 2-ethylhexylacrylate, and butylacrylate. Concrete examples of the monomers constituting the hard material may include methacrylic acid ester such as methylmethacrylate, ethylmethacrylate, propylacrylate, and butylacrylate. The soft monomer or a mixture of the soft monomer and the hard monomer is polymerized and hardened to produce the IOL base material. It is to be noted that the optic part 101 can be produced by a cast molding method achieved by putting the IOL base material (monomer) before polymerization into a predetermined mold form and polymerizing and hardening the base material or a lathe cutting method achieved by cutting a polymerized and hardened IOL base material into a lens form.

Each of the support parts 103 has an open loop shape including one end (a base end) joined to the optic part 101 and the other end (a distal end) formed as a free end. Each support part 103 is made of resin such as PMMA (polymethyl methacrylate) and polypropylene. Each support part 103 is connected to the optic part 101 with a forward inclination at a predetermined angle (e.g., about 0° to 10°) as shown in FIG. 1B.

The IOL 100 in this embodiment is one example of a three-piece type IOL produced of the optic part and the support parts individually produced and joined to each other, but the IOL it not limited to such configuration. For example, the IOL may be of one-piece type including an optic part and supports ports integrally formed of the same material. If the optic part 101 has high adhesive properties, the surfaces of the lens adhere to each other when folded and the optic part 101 is not easily unfolded in the eye. To avoid such defect, the optic part 101 is subjected to a surface treatment such as a plasma treatment to reduce the adhesive properties of the optic part 101.

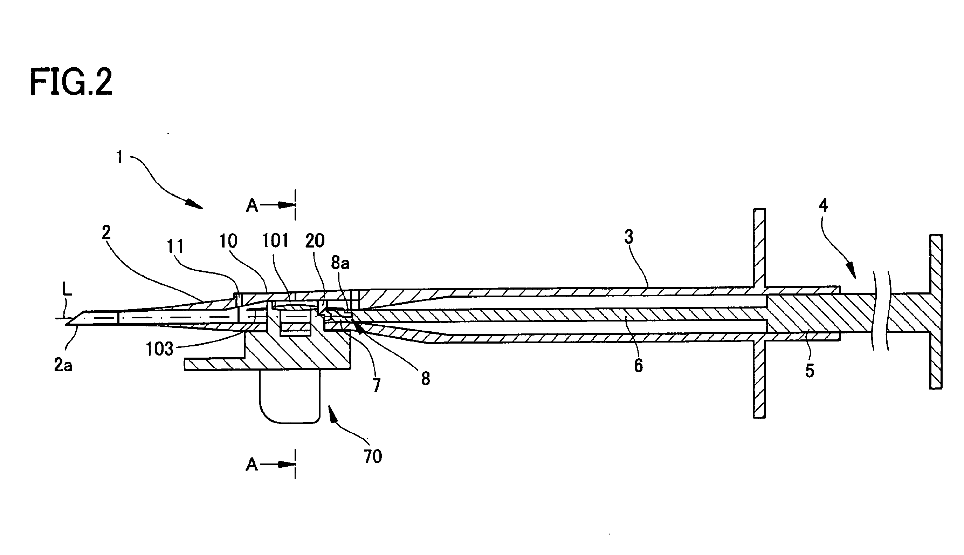

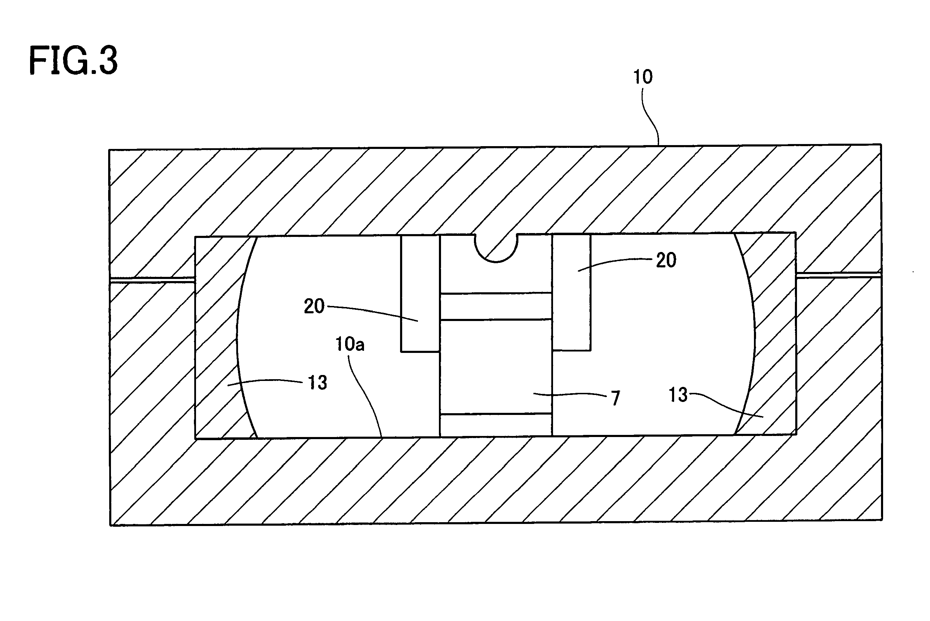

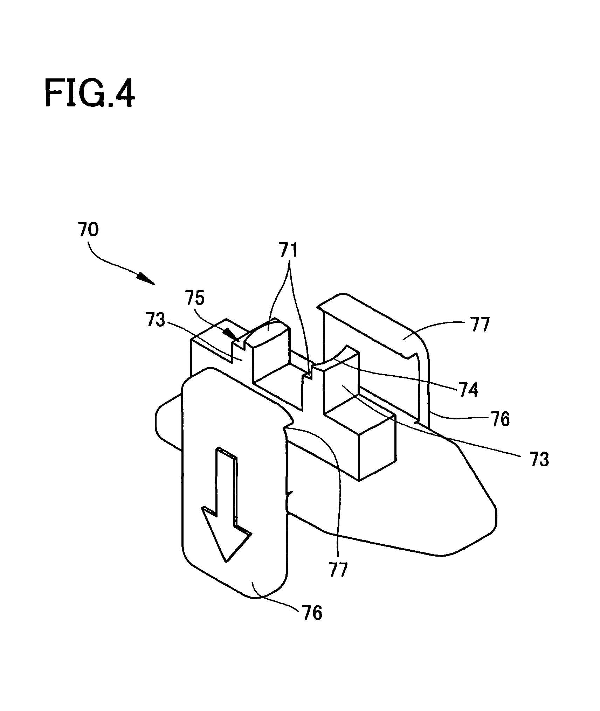

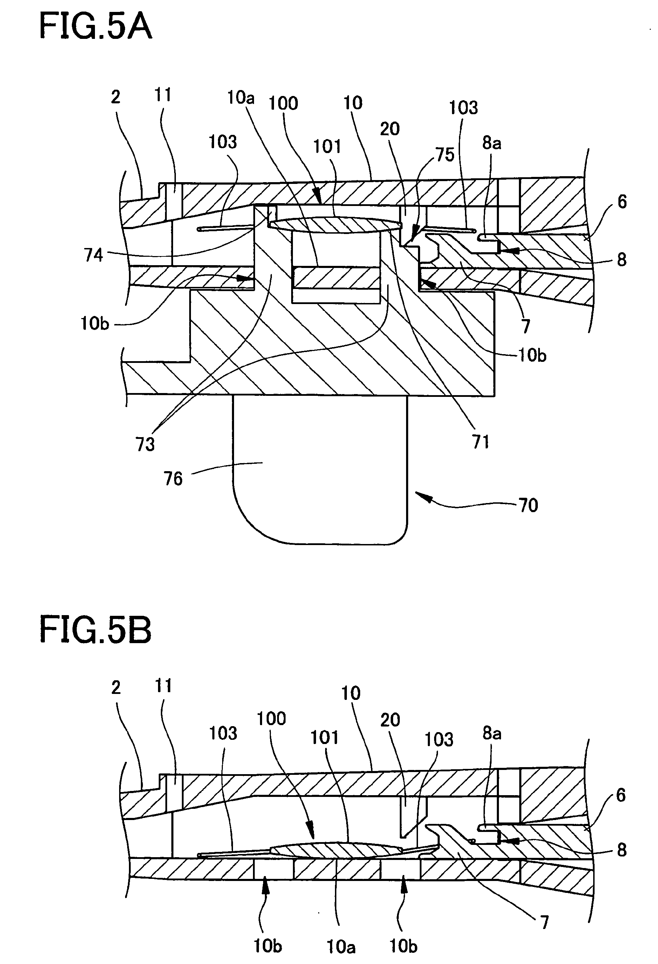

The configuration of the IOL injection instrument (hereinafter, simply referred to as an "injector") 1 in the present embodiment will be explained below. FIG. 2 is a schematic sectional view of the injector 1. FIG. 3 is a schematic view to explain centering by centering part 20, showing a sectional view of a setting part (a housing) 10 taken along a line A-A, from which a storage member (a storage part) 70 is removed. FIG. 4 is a perspective view of the storage member 70. FIG. 5A and 5B are enlarged views showing the setting part 10 and its surroundings in sectional views to explain a release motion of the storage member 70.

As shown in FIG. 2, the injector 1 mainly includes, in the order of injection into the eye, an injection part 2 to be injected in an incision formed in the eye, a setting part 10 provided at a rear end of the injection part 2 and designed to mount therein the IOL 100, an outer sleeve 3 continuous with the setting part 10 and formed with a cylindrical inner wall, and a rod-like push-out member 4 placed through the inside of the outer sleeve 3 and slidable to push out the IOL 100 through the injection part 2. The storage member 70 is removably attached to a lower part of the setting part 10 of the injector 1 so that the optic part 101 is placed above and out of the movement axis when the storage member 70 is positioned upside and the optic part 101 is placed on the movement axis when the storage member 70 is positioned downside. The storage member 70 serves to place and store the IOL 100 (the optic part 101) above a push-out axis (a movement axis) in the main unit.

In the present embodiment, the injector 1 is an assembly of components molded of resin and others as an instrument of a disposable type which is to be thrown away after one use. The material thereof may include polypropylene and polycarbonate.

The injection part 2 is a tapered hollow member whose diameter is smaller than that of the rear outer sleeve 3 and decreases toward a front end 2a in order to facilitate injection of the IOL in a folded state without widening the incision formed in the eye more than necessary. Furthermore, the injection part 2 has an almost circular cross section (a section perpendicular to an axis L). The injection part 2 is formed, at its rear end, with side walls 13 (see FIG. 3) for rolling (folding) the optic part 101 when pushed forward. The side walls 13 are placed right and left relative to the optic part 101 and an interval between the opposite side walls 13 is narrower toward the front end 2a. Accordingly, the optic part 101 is rolled or folded by the storage member 2 and the side walls 13 as the optic part 101 is moved toward the front end 2a. The IOL 100 is fed from the setting part 10 through the front end 2a and injected into the patient's eye.

The push-out member 4 includes an axial base part 5 provided with a press part to be pressed by an operator and configured to prevent deflection during movement inside the outer sleeve 3, a push rod 6 extending forward from a front end of the axial base part 5 and having a diameter smaller than that of the axis base part 5, a front end portion 7 formed at a front end of the push rod 6 to contact with and hold the optic part 101, and a receipt portion 8 provided in a recessed form at the rear of the front end portion 7 to receive a distal end portion of the rear support part 103. The push-out member 4 is attached to be movable forward and backward in a front-rear direction (in a movement axis L direction) in the outer sleeve 3. The front end portion 7 is formed with a groove (a guide groove) shaped to catch the optic part 101 so as to catch and push the optic part 101. The push rod 6 and the front end portion 7 are made of a relatively soft resin material to prevent damage to the IOL 100. The length of the push rod 6 is determined to be enough long to allow the front end portion 7 to sufficiently protrude from the front end of the storage member 2 when the push-out member 4 is pushed forward in the outer sleeve 3. The receipt portion 8 is a recess formed on the push rod 6 and in such a place as to receive the rear support part 103 extending from the optic part 101 caught by the front end portion 7, without allowing the rear support part 103 to unbend. The receipt portion 8 further includes an overhang portion 8a on an upper side to prevent the support part 103 received in the receipt portion 8 from slipping upward out of the receipt portion 8.

The setting part 10 is formed with a setting surface 10a for mounting thereon the optic part 101 when the IOL 100 is to be pushed out.

Above the setting part 10, a centering part 20 is provided to allow the push rod 6 to pass through for centering of the push rod 6. The centering part 20 shown in FIG. 3 includes a pair (two) of columnar members placed at a distance equal to or slightly wider than a lateral width of the push rod 6 to prevent lateral deflection of the push rod 6. The centering part 20 is formed to extend (protrude) downward from an upper wall (a ceiling) of the setting part 10 by a predetermined length. The centering part 20 is formed inside the setting part 10 so as to be located in a space created between the optic part 101 and the distal end of the rear support part 103 when the IOL 100 to be pushed out is mounted on the setting surface 10a. The centering part 20 is preferably formed in a place as close to the optic part 101 as possible. The centering part 20 has only to be configured to prevent deflection of the push rod 6 in the right and left direction in FIG. 3 to perform centering. The centering part 20 includes at least two members.

The centering part 20 is located between the optic part 101 and the distal end of the rear support part 103 while the IOL 100 to be pushed out is mounted on the setting surface 10a. Accordingly, the support part 103 has to be released from the centering part 20 during movement of the IOL 100. In the present embodiment, therefore, a predetermined clearance is provided between an end (a lower end) of the centering part 20 and a bottom surface of the setting part 10. This clearance is narrower than the thickness of the optic part 101 to restrict backward movement of the optic part 101 and sufficiently wider than the diameter of the support part 103 to release (move forward) the support part 103. A surface (a front wall surface) of the centering part 20 which will contact with a side surface (an edge) of the optic part 101 is formed as a predetermined curved surface substantially conforming to a circular shape of the optic part 101 to adequately restrict the backward movement of the optic part 101.

The centering part 20 includes a configuration for guiding the movement (release) of the rear support part 103 during a push-out operation. In the present embodiment, the centering part 20 guides the support part 103 received in the receipt portion 8 toward the clearance between the lower end of the centering part 20 and the bottom surface of the setting part 10 during the push-out operation. To be concrete, the centering part 20 is formed with a slant surface made by cutting an end portion obliquely downward in a movement direction. This slant surface is formed up to at least a height corresponding to the position of the rear support part 103 when the optic part 101 is mounted on the setting part 10a.

During the push-out operation, such configuration of the centering part 20 guides the advancing support part 103 downward along the slant surface formed as above, thereby appropriately releasing the support part 103 forward through the clearance between the centering part 20 and the bottom surface of the setting part 10.

Furthermore, as shown in FIG. 2, the setting part 10 is formed, in its ceiling, with an injection port 11 through which a lubricant such as a viscoelastic material is injected. The injection port 11 is formed through the wall (the ceiling) of the setting part 10 and with a diameter allowing insertion of an injection needle for the viscoelastic material and others.

The storage member 70 is explained below. The storage member 70 includes holding stands 73 insertable in the setting part 10 through holes 10b formed in a lower portion (the bottom wall) of the setting part 10 to hold the IOL 100 on upper surfaces 71 and grip parts 76 serving to fixedly attach the storage member 70 to the main unit of the injector 1 and also as a grip to be gripped by a user.

The holding stands 73 are arranged in pair in a front-rear direction of the storage member 70. The upper surfaces 71 of the holding stands 73 support thereon a part of a peripheral portion of a lower surface of the optic part 101. Each upper surface 71 has a shape conforming to the curved surface of the optic part 101 and, in the present embodiment, has a curve along a curvature radius of the optic part 101.

The length (height) of the holding stands 73 is determined to be long enough to place the optic part 101 in a position off the axis L (off the movement axis) and prevent the optic part 101 placed on the upper surfaces 71 from contacting the inside (the ceiling) of the setting part 10 while the storage member 70 is fixed to the setting part 10 and the optic part 101 is placed on the upper surfaces 71. The optic part 101 placed on the upper surfaces 71 is located sufficiently above the movement axis of the push rod 6, so that the viscoelastic material is easily injected in the setting part 10 and applied to the optic part 101. The holding stand 73 located on a front side (closer to the injection part 2) is provided with a wall 74 extending higher than the upper surface 71 to restrict forward movement of the optic part 101. The height of the wall 74 is preferably determined to be higher than the edge thickness of the optic part 101 placed on the upper surface 71. The wall 74 restricts the forward movement of the optic part 101. Furthermore, a surface of the wall 74 that will contact with the optic part 101 has a shape (a curved surface) conforming to the circular shape of the optic part 101, thereby restricting rightward and leftward movements of the optic part 101 (in a direction perpendicular to the movement axis L). The wall 74 has only to be provided in at least one of the holding stands 73.

On the other hand, the rear holding stand 73 is provided, at its rear side, with a cutout 75 located lower than the upper surface 71. The cutout 75 is formed to allow the centering part 20 located just behind the optic part 101 to serve as a wall for restricting backward movement of the optic part 101 without interfering with the holding stand 73 (see FIG. 5A).

Even when the optic part 101 is subjected to a surface treatment, its adhesive properties cannot be completely removed. In the present embodiment, therefore, the optic part 101 is allowed to adhere to the upper surfaces 71 of the holding stands 73 by taking advantage of the remaining adhesive properties of the optic part 101 which could not be completely removed. Thus, the optic part 101 is fixedly held with stronger force than when simply placed. Accordingly, at least the columnar members (the holding stands) are made of a resin material and in a shape easy to adhere to the adhesive properties of acrylate resin in order to allow the optic part 101 to easily adhere to the upper surfaces 71. The resin material for the holding stands 73 may include polycarbonate, polypropylene, polyethylene, PMMA, POM, ABS resin, etc. In particular, polycarbonate has good adhesive properties and therefore the storage member 70 is preferably made of polycarbonate by injection molding. In the injection molding, the surface roughness of a corresponding mold is made as smaller as possible, so that of a resultant storage member 70 can have the upper surfaces 71 as mirror surfaces. This enables more firm adhesion with the optic part 101. Such surface roughness is predetermined 6.3 or less in arithmetical mean roughness Ra so that the optic part 101 is allowed to adhere to the upper surfaces 71 by the viscosity of the optic part 101 and the surface roughness of the upper surfaces 71. If the surface roughness exceeds Ra 6.3, the optic part 101 can adhere to the surfaces 71 but adhesive strength is low, which may improperly cause detachment of the optic part 101. If lower than Ra 6.3, on the other hand, appropriate adhesive strength is attained. However, Ra is not required to be less than 0.012 in view of cost-benefit performance.

The holding stand is not limited to the above paired configuration arranged in the front-rear direction. The holding stand has only to hold (adhere) the optic part on the upper surface and may be provided as a single member or three or more members.

An upper part of each grip 76 is formed with a hook 77 for fixing the storage member 70 to an outer wall side surface of the setting part 10. When a user pinches the grips 76 and moves them in an arrow direction (downward) in the figure, pulling the holding stands 73 and others out of the setting part 10. The optic part 101 larger than each hole 10b is thus retained on the setting surface 10a and the storage member 70 is detached from the setting part 10. Accordingly, the IOL 100 is placed in an initial position where the IOL 100 is to be pushed.

Push-out operation of the IOL 100 in the injector 1 having the above configuration will be explained below. Herein, an example is described in which the injector 1 with the IOL 100 being mounted on (the upper surfaces 71 of the holding stands 73 of) the storage member 70 fixed to the setting part 10 is shipped from a factory and used by an operator. This state corresponds to a state shown in FIG. 5A. The optic part 101 adhered to the holding storage member 70 is less displaced due to impacts or vibrations during carriage or transport of the injector 1.

The operator then injects a viscoelastic material into the setting part 10 through the injection port 11 by use of an injector such as a syringe. At that time, the optic part 101 is placed out of the axis by the storage member 70 and a part of the optic part 101 is held on the upper surfaces 71. Accordingly, the viscoelastic material is distributed over a front surface (downward in the drawing sheet) and a rear surface (upward in the drawing sheet) of the optic part 101. When the operator pinches and moves the grips 76 of the storage member 70 downward, the upper surfaces 71 are also moved downward. Simultaneously, the optic part 101 adhered on the upper surfaces 71 is moved downward together with the storage member 70.

In association with downward movement of the optic part 101, the viscoelastic material existing on the front surface (a lower surface) of the optic part 101 is pressed and spread out in the setting part 10. At that time, the upper surfaces 71 and the optic part 101 adhere to each other, so that the optic part 101 is moved downward without detaching from the storage member 70. This motion causes the viscoelastic material to be distributed into the setting part 10 and applied or coated onto the optic part 101.

When the optic part 101 is moved downward into contact with the setting surface 10a, the optic part 101 is retained on the setting surface 10a and placed on the axis L. Furthermore, the upper surfaces 71 are moved downward in association with movement of the storage member 70 pulled down. By a downward force on the storage member 70, the adhesion of the upper surfaces 71 to the optic part 101 is released (detached), and only the holding stands 73 are pulled out of the setting part 10. The storage member 70 is thus detached from the setting part 10 (a state shown in FIG. 5B). At that time, the rear support part 103 is also moved downward in association with movement of the optic part 101 and then is received in the receipt portion 8. In this way, the optic part 100 is placed on the axis L along which the optic part 100 is to be pushed out.

The operator starts an operation to push the IOL 100 out of the injector 1 placed in a usable state. When the push-out member 4 is pushed, advancing the front end portion 7, the optic part 101 is caught in the front end portion 7. The push rod 6 is then fit in the centering part 20 whereby the push rod 6 (the front end portion 7) is centered. The centered push rod 6 is moved ahead without deflecting in the right and left directions. The optic part 101 is folded by the side walls 13 and, as moved toward the front end, the optic part 101 is further rolled into a smaller size by the internal shape of the injection part 2. At that time, as the optic part 101 advances, the support part 103 received in the receipt portion 8 comes into contact with the slant surface of the centering part 20 and slides downward. The support part 103 reaching a lower end of the centering part 20 goes under the centering part 20 to the front side thereof. Thus, the support part 103 can be moved forward without interfering with the centering part 20. Furthermore, the support part 103 can be moved forward without becoming unbent by the centering part 20.

The operator inserts the front end 2a of the injection part 2 into the previously formed incision of the patient's eye and injects the optic part 100 into the eye. Since the rear support part 103 is being received in a bent state in the receipt portion 8, the shape of the rear support part 103 will not be largely changed after injection in the eye. Accordingly, the rear support part 103 is less apt to return and swing and hence touch a posterior capsule and others.

As above, the IOL 100 is injected in the patient's eye. In this way, the IOL having the adhesive properties and the storage member to which the IOL adheres can cause the viscoelastic materials or the like to be appropriately supplied to the vicinity of the IOL and applied to the overall IOL. Furthermore, the IOL can be placed in a push-out position without displacement. The centering part can serve to appropriately push out the IOL. It is further possible to prevent deflection of the push rod and hence appropriately push out the IOL.

In the aforementioned embodiment, the centering part is formed to extend downward from the ceiling of the setting part. The centering part has only to be configured to allow centering but not to disturb forward movement of the support part. Preferably, the centering part is configured to restrict movement of the IOL in a storage state. For instance, it may be formed to extend upward from the setting surface or extend laterally from a side wall. The centering part is preferably placed as close to the optic part as possible. However, the centering part has only to be able to appropriately center without limiting to a configuration placed just behind the optic part.

In the aforementioned embodiment, the push rod is provided with the receipt portion for receiving the support part. However, the push rod has not always to include the receipt portion. In this case, during the push-out operation, the rear support part contacts with the centering part and is unbent by the centering part. When the push-out operation further advances, the distal end of the support part goes ahead of the centering part from the side of the centering part. As above, the rear support part can be moved forward even if no receipt portion is provided. In such a case, therefore, the centering part may be configured to join the upper side (the ceiling) and the lower side (the bottom) of the setting part.

In the aforementioned embodiment, the IOL stored out of the movement axis is put on the movement axis by a pull-out operation of the storage member and then the push rod is centered by the centering part. As an alternative, the push rod may be centered without use of the storage member. In this case, the optic part of the IOL does not have to be viscous. For instance, an IOL made of a low-viscous material such as silicone may be put on the setting surface and subjected to the push-out operation.

In the aforementioned embodiment, the rear holding stand of the storage member is formed with the cutout to allow the centering part provided in the setting part to restrict backward movement of the optic part. As an alternative, the rear holding stand may be provided with a wall similar to the front holding stand so that the storage member restricts forward and backward movements of the optic part.

### Reference Signs List

- 1: Intraocular lens injector
- 2: Injection part
- 3: Outer sleeve
- 4: Push-out member
- 20: Centering part
- 70: Storage member
- 71: Upper surface
- 73: Holding stand
- 100: Intraocular lens
- 101: Optic part
- 103: Support part

## Claims

1. An intraocular lens injection instrument (1) for storing a soft intraocular lens (IOL) (100) including an optic part (101) made of acrylate resin having viscosity in a housing (10) of the instrument (1), and feeding out the IOL (100) from the housing (10) through a front end (2a) of the instrument by use of a push rod (6) of the instrument (1) to inject the IOL (100) into a patient's eye,
whereby
the instrument (1) comprises a storage part (70) provided with a pair of holding stands (73) arranged opposite to each other in a front-rear direction along a movement axis (L) of the push rod (6) to hold the optic part (101), the holding stands (73) including upper surfaces (71) on which the optic part (101) is to be placed and having a predetermined height to place the optic part (101) above and out of the movement axis (L) during storage,
the upper surfaces (71) have a surface roughness of arithmetical mean roughness Ra 6.3 or less so that the optic part (101) is allowed to adhere to the upper surfaces (71) by the viscosity of the optic part (101) and the surface roughness of the upper surfaces (71), and
the storage part (70) is vertically movably attached to the housing (10) so that the optic part (101) is placed above and out of the movement axis (L) when the storage part (70) is positioned upside and the optic part (101) is placed on the movement axis (L) when the storage part (70) is positioned downside.

2. The intraocular lens injection instrument (1) according to claim 1, wherein the upper surfaces (71) are mirror surfaces.

3. The intraocular lens injection instrument (1) according to claim 1 or 2, wherein the holding stands (73) are made of polycarbonate or polypropylene.

4. The intraocular lens injection instrument (1) according to one of claims 1 to 3, wherein at least one of the holding stands (73) includes a wall (74) higher than the upper surfaces (71) and higher than a thickness of an edge of the optic part (101) placed on the upper surfaces (71), the wall (74) having a curved surface almost conforming to a circular shape of the optic part (101) to come into contact with the optic part (101).

## Patentansprüche

1. Ein Einspritzinstrument (1) für Intraokularlinsen zum Speichern einer weichen Intraokularlinse (IOL) (100) einschließlich eines aus einem Acrylatharz mit Viskosität gebildeten optischen Teils (101) in einem Gehäuse (10) des Instruments (1), und zum Herausschieben der IOL (100) aus dem Gehäuse (10) durch ein vorderes Ende (2a) des Instruments durch Verwendung eines Schubstabes (6) des Instruments (1), um die IOL (100) in das Auge eines Patienten einzuspritzen,
wobei
das Instrument (1) einen Speicherabschnitt (70) umfasst, der mit einem Paar Haltesockeln (73) versehen ist, die einander gegenüberliegend in einer vom-hinten-Richtung entlang einer Bewegungsachse (L) des Schubstabs (6) angeordnet sind, um das optische Teil (101) zu halten, wobei die Haltesockel (73) obere Oberflächen (71) aufweisen, auf welchen das optische Teil (101) zu platzieren ist, und die eine vorbestimmte Höhe besitzen, um das optische Teil (101) oberhalb und außerhalb der Bewegungsachse (L) während des Speicherns zu platzieren,
die oberen Oberflächen (71) eine Oberflächenrauhigkeit einer arithmetisch gemittelten Rauhigkeit Ra von 6,3 oder weniger besitzen, so dass das optische Teil (101) in der Lage ist, an den oberen Oberflächen (71) aufgrund der Viskosität des optischen Teils (101) und der Oberflächenrauhigkeit der oberen Oberflächen (71) anzuhaften, und
der Speicherabschnitt (70) vertikal beweglich an dem Gehäuse (10) angebracht ist, so dass das optische Teil (101) oberhalb und außerhalb der Bewegungsachse (L) platziert ist, wenn der Speicherabschnitt (70) auf der oberen Seite positioniert ist, und das optische Teil (101) auf der Bewegungsachse (L) platziert ist, wenn der Speicherteil (70) auf der unteren Seite positioniert ist.

2. Das Einspritzinstrument (1) für Intraokularlinsen gemäß Anspruch 1, wobei die oberen Oberflächen (71) Spiegeloberflächen sind.

3. Das Einspritzinstrument (1) für Intraokularlinsen gemäß Anspruch 1 oder 2, wobei die Haltesockel (73) aus Polykarbonat oder Polypropylen gebildet sind.

4. Das Einspritzinstrument (1) für Intraokularlinsen gemäß einem der Ansprüche 1 bis 3, wobei wenigstens einer der Haltesockel (73) eine Wand (74) aufweist, die höher ist als die oberen Oberflächen (71), und höher ist als eine Dicke einer Kante des optischen Teils (101), das auf den oberen Oberflächen (71) platziert ist, wobei die Wand (74) eine gekrümmte Oberfläche besitzt, die nahezu mit einer Kreisform des optischen Teils (101) übereinstimmt, um in Kontakt mit dem optischen Teil (101) zu kommen.

## Revendications

1. Instrument d'injection de lentille intraoculaire (1) pour stocker une lentille intraoculaire souple (IOL) (100), comprenant une partie optique (101) faite d'une résine acrylique ayant une viscosité, dans un boîtier (10) de l'instrument (1), et faire sortir la IOL (100) du boîtier (10) par une extrémité avant (2a) de l'instrument à l'aide d'une tige de poussée (6) de l'instrument (1) afin d'injecter la IOL (100) dans l'oeil d'un patient,
moyennant quoi
l'instrument (1) comprend une partie de stockage (70) prévue avec une paire de socles de support (73) agencés à l'opposé l'un de l'autre dans une direction avant-arrière le long d'un axe de déglacement (L) de la tige de poussée (6) pour supporter la partie optique (101), les socles de support (73) comprenant des surfaces supérieures (71) sur lesquelles la partie optique (101) doit être placée et ayant une hauteur prédéterminée pour placer la partie optique (101) au-dessus et hors de l'axe de déplacement (L) pendant le stockage,
les surfaces supérieures (71) ont une rugosité de surface dont la moyenne arithmétique de rugosité Ra est de 6,3 ou moins de sorte que la partie optique (101) est autorisée à adhérer sur les surfaces supérieures (71) par la viscosité de la partie optique (101) et la rugosité de surface des surfaces supérieures (71), et
la partie de stockage (70) est fixée de manière verticalement mobile sur le boîtier (10) de sorte que la partie optique (101) est placée au-dessus et hors de l'axe de déplacement (L) lorsque la partie de stockage (70) est positionnée vers le haut et la partie optique (101) est placée sur l'axe de déplacement (L) lorsque la partie de stockage (70) est positionnée vers le bas.

2. Instrument d'injection de lentille intraoculaire (1) selon la revendication 1, dans lequel les surfaces supérieures (71) sont des surfaces de miroir.

3. Instrument d'injection de lentille intraoculaire (1) selon la revendication 1 ou 2, dans lequel les socles de support (73) sont réalisés à partir de polycarbonate ou de polypropylène.

4. Instrument d'injection de lentille intraoculaire (1) selon l'une quelconque des revendications 1 à 3, dans lequel au moins l'un des socles de support (73) comprend une paroi (74) plus haute que les surfaces supérieures (71) et plus haute qu'une épaisseur d'un bord de la partie optique (101) placée sur les surfaces supérieures (71), la paroi (74) ayant une surface incurvée se conformant presque à une forme circulaire de la partie optique (101) pour venir en contact avec la partie optique (101).
